(19) Europäisches Patentamt / European Patent Office / Office européen des brevets

(11) **EP 2 457 561 A1**

(12) **EUROPEAN PATENT APPLICATION**
published in accordance with Art. 153(4) EPC

(43) Date of publication:
**30.05.2012   Bulletin 2012/22**

(21) Application number: **10797110.3**

(22) Date of filing: **05.07.2010**

(51) Int Cl.:
*A61K 9/20* (2006.01)     *A61K 31/426* (2006.01)
*A61K 47/08* (2006.01)     *A61K 47/10* (2006.01)
*A61K 47/14* (2006.01)     *A61K 47/26* (2006.01)
*A61K 47/36* (2006.01)     *A61K 47/38* (2006.01)
*A61K 47/44* (2006.01)     *A61P 1/04* (2006.01)

(86) International application number:
**PCT/JP2010/061430**

(87) International publication number:
**WO 2011/004799 (13.01.2011 Gazette 2011/02)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority:  **06.07.2009   JP 2009159804**
              **02.11.2009   JP 2009251715**

(71) Applicant: **Kyorin Pharmaceutical Co., Ltd.
Tokyo 101-8311 (JP)**

(72) Inventors:
• **FUKUDA, Mamuro
Tokyo 101-8311 (JP)**
• **GOTO, Akinori
Shimotsuga-gun, Tochigi (JP)**

(74) Representative: **Harmsen, Dirk
Vossius & Partner
Siebertstrasse 4
81675 München (DE)**

(54) **TABLET HAVING HOLLOW STRUCTURE**

(57)     A water floatable tablet, which has a hollow structure having a cavity part in the center section and comprises at least one filler selected from sugar alcohols, sugars, cellulose derivatives and starches and a component which exhibits a hydrophobic effect.

*Fig. 3*

20x  500 μm  WD:13.6mm   2kV 2009/03/18 14:04:49 S

EP 2 457 561 A1

## Description

Technical Field

The present invention relates to a tablet which has a hollow structure having a cavity part in the center section and is floatable in water and a production method of said tablet. Particularly, the invention relates to a tablet which remains in the stomach and also has a sustained release effect.

Background of the Invention

Gastric retention type tablets are known as tablets which sustainably release drugs in the stomach. As these gastric retention type tablets, a gel swelling type tablet which remains in the stomach by swelling in the stomach (Patent Literatures 1 to 9), a bubbling type tablet which is floatable by foaming due to the gastric acid (Patent Literatures 10 to 13) and a gastric floating type tablet which is floatable in the stomach by using material having a low density (Patent Literatures 14 and 15) or creating a porous void (Patent Literatures 16 and 17) are known. In addition, in the case of granules, pharmaceutical preparations which are floatable in the stomach by arranging a cavity part in each granule (Patent Literatures 18 to 20, Non-patent Literature 1) are known.

In the case of other than the oral administration type pharmaceutical preparations, a technique for using a porous composition is used in agricultural chemicals as a technique for floating it on the water surface (Patent Literatures 21 and 22). Also, a technique for using a foaming composition is used for bathing agents (Patent Literature 23).

Citation List

Patent Literature

Patent Literature 1 : JP-A-2007-131591
Patent Literature 2 : JP-T-2005-532985
Patent Literature 3 : JP-A-2002-370970
Patent Literature 4 : W095/5809
Patent Literature 5 : JP-A-2005-132803
Patent Literature 6 : JP-T-2001-500879
Patent Literature 7 : JP-T-2008-528636
Patent Literature 8 : JP-A-6-24959
Patent Literature 9 : JP-A-2009-40787
Patent Literature 10 : JP-A-62-283919
Patent Literature 11 : JP-A-62-195323
Patent Literature 12 : JP-A-62-207209
Patent Literature 13 : JP-T-2009-541777
Patent Literature 14 : JP-A-1-16715
Patent Literature 15 : JP-A-2005-112825
Patent Literature 16 : JP-A-61-43108
Patent Literature 17 : W091/6281
Patent Literature 18 : JP-A-64-30
Patent Literature 19 : JP-A-1-224311
Patent Literature 20 : JP-A-2-250822
Patent Literature 21 : JP-A-5-255067
Patent Literature 22 : WO00/040085
Patent Literature 23 : JP-A-10-87477

Non-patent Literature

Non-patent Literature 1 : European Journal of Pharmaceutics and Biopharmaceutics, Vol.57, 235-243 (2004)

Summary of Invention

Technical Problems

A problem to be solved by the invention is to obtain a tablet having high general purpose property and allowing sustained release of a drug by floating in water or retaining in the stomach, which can be prepared by a convenient production method.. Further, it is to establish a technique applicable to various tablets which are floatable in water.

Solution to Problem

The present inventors have conducted intensive studies on a tablet which can be produced conveniently, has high general purpose property and can be floatable in water. As a result, it was found that a hollow tablet which can be floatable in water can be obtained by preparing a core part containing a sublimation solid such as menthol, forming a crust containing a filler on the outside of the core part in such a manner that the core part is positioned at the center section and then removing the sublimation solid contained in the core part by heating, and penetrating a hydrophobic component, and thus the inventors accomplish the invention.
That is, the invention includes the following inventions.

1. A water floatable tablet, which has a hollow structure having a cavity part in the center section and comprises at least one filler selected from sugar alcohols, sugars, cellulose derivatives and starches and a component which exhibits a hydrophobic effect.
2. A water floatable tablet, which is obtainable by a production method comprising at least the following steps (1) to (4):

(1) a step of preparing a core part comprising a sublimation solid;
(2) a step of preparing a dry coated tablet by forming a crust comprising at least one or more fillers selected from sugar alcohols, sugars, cellulose derivatives and starches on the outside of the core part in such a manner that the core part obtained by the step (1) is positioned at the center section;
(3) a step of obtaining a tablet having a hollow structure by removing the sublimation solid comprised in the core part by heating the dry coated tablet obtained in the step (2); and
(4) a step of immersing the tablet having a hollow structure obtained in the step (3) in a component which exhibits a hydrophobic effect.

3. The tablet described in the aforementioned item 1 or 2, wherein the component which exhibits a hydrophobic effect is a component which shows the hydrophobic effect in the stomach.
4. The tablet described in any one of the aforementioned items 1 to 3, which has a density of 1 g/cm$^3$ or less.
5. The tablet described in any one of the aforementioned items 1 to 4, which comprises a main drug component is comprised in a part other than the cavity part.
6. The tablet described in any one of the aforementioned items 1 to 5, which has a sustained release effect.
7. The tablet described in any one of the aforementioned items 1 to 6, wherein the filler is at least one substance selected from mannitol, crystalline cellulose, lactose and a mixture thereof.
8. The tablet described in any one of the aforementioned items 1 to 7, wherein the component which exhibits a hydrophobic effect is at least one of a higher alcohol and a higher fatty acid glycerin ester.
9. The tablet described in any one of the aforementioned items 1 to 8, wherein the component which exhibits a hydrophobic effect is at least one substance selected from stearyl alcohol, cetyl alcohol, hydrogenated castor oil and stearic acid monoglyceride.
10. A method for producing a water floatable tablet having a hollow structure, which comprises at least the following steps:

(1) a step of preparing a core part comprising a sublimation solid;
(2) a step of preparing a dry coated tablet by forming a crust comprising at least one or more fillers selected from sugar alcohols, sugars, cellulose derivatives and starches on the outside of the core part in such a manner that the core part obtained by the step (1) is positioned at the center section;
(3) a step of obtaining a tablet having a hollow structure by removing the sublimation solid comprised in the core part by heating the dry coated tablet obtained in the step (2); and
(4) a step of immersing the tablet having a hollow structure obtained in the step (3) in a component which exhibits a hydrophobic effect.

11. The production method described in the aforementioned item 10, wherein the component which exhibits a hydrophobic effect is a component which shows the hydrophobic effect in the stomach.

12. The production method described in the aforementioned item 10 or 11, wherein the sublimation solid is at least one of terpenes and an aromatic hydrocarbon having subliming property.

13. The production method described in the aforementioned item 12, wherein the terpenes are at least one substance selected from menthol, thymol and camphor.

14. The production method described in any one of the aforementioned items 10 to 13, wherein a main drug component is comprised in the crust part in the step (2).

15. The production method described in any one of the aforementioned items 10 to 14, wherein the filler is at least one substance selected from mannitol, crystalline cellulose, lactose and a mixture thereof.

16. The production method described in any one of the aforementioned items 10 to 15, wherein the component which exhibits a hydrophobic effect is at least one of a higher alcohol and a higher fatty acid glycerin ester.

17. The production method described in any one of the aforementioned items 10 to 16, wherein the component which exhibits a hydrophobic effect is at least one substance selected from stearyl alcohol, cetyl alcohol, hydrogenated castor oil and stearic acid monoglyceride.

18. A method for retaining a tablet in the stomach by using the tablet described in any one of the aforementioned items 1 to 9.

19. A method for sustainably releasing a drug in the stomach by using the tablet described in any one of the aforementioned items 1 to 9.

Advantageous Effects of Invention

The tablet of the invention can be floatable in water since it has a hollow structure and also comprises a component which exhibits a hydrophobic effect. When a drug is contained in the tablet of the invention, the tablet can be floatable in water or retained in the stomach and also can exhibit a sustained release effect of the main drug component. In addition, the tablet of the invention having a hollow structure can be prepared very conveniently as compared with the conventional techniques.

Brief Description of the Drawings

[Fig. 1] Fig. 1 shows a change in mass when the dry coated tablets of Reference Examples 1 to 5 were heated at 80°C in an oven in Test Example 1.

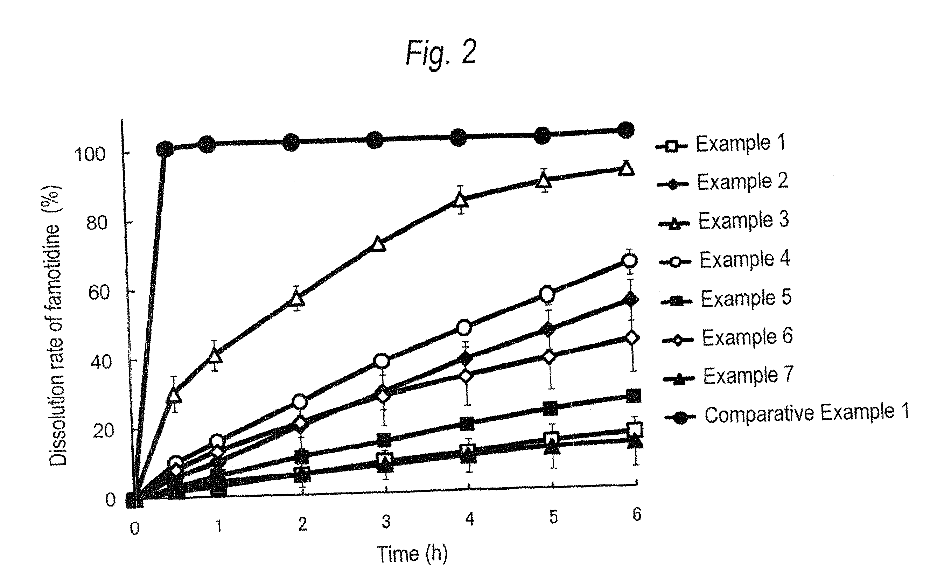

[Fig. 2] Fig. 2 shows a result of dissolution tests of Examples 1 to 7 and Comparative Example 1 in Test Example 2.

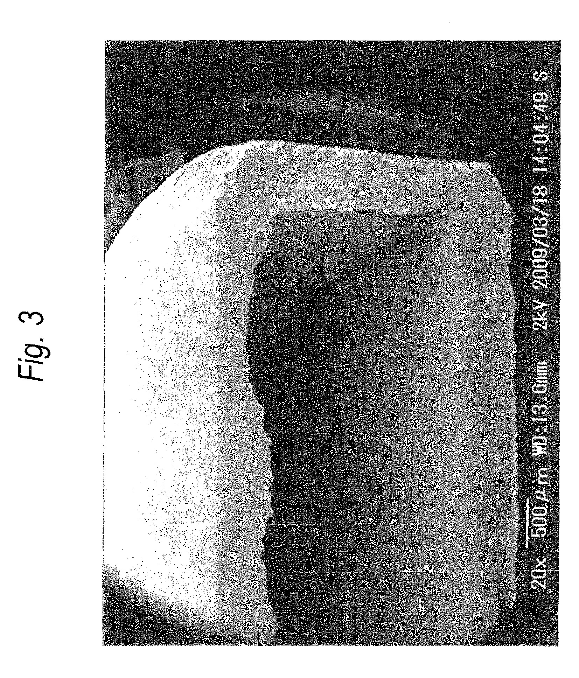

[Fig. 3] Fig. 3 is a photograph of inner structure of the hollow tablet concerned in the invention which was taken using a scanning electron microscope.

Description of Embodiment

The invention is a water floatable tablet which has a hollow structure having a cavity part in the center section and comprises at least one filler selected from sugar alcohols, sugars, cellulose derivatives and starches and a component which exhibits a hydrophobic effect.

The tablet of the invention has a hollow structure having a cavity part in the center section. By having said hollow structure, the tablet of the invention becomes floatable in water. The tablet of the invention having the hollow structure is distinguished from the conventional foaming type tablets (JP-A-62-283919, JP-A-62-195323, JP-A-62-207209 and JP-T-2009-501777) and the tablets having porous void (JP-A-61-43108 and WO91/6281).

Size of the cavity part can be optionally adjusted in such a manner that the tablet strength can be ensured and also that it has a size suitable for being floatable in water. In addition, the number of cavity parts is not always one but may be two or more.

The cavity part is positioned at the center of the tablet from the viewpoint of tablet strength, but it is not always required that the follow portion is positioned precisely at the center of the tablet and it can be optionally adjusted within such a range that the tablet strength can be ensured.

It is preferable that the aforementioned size of cavity part is optionally adjusted in such a manner that the tablet becomes floatable in water. In this case, in order to make the tablet floatable in water, density of the whole tablet is preferably 1 $g/cm^3$ or less, preferably 0.95 $g/cm^3$ or less and particularly preferably 0.90 $g/cm^3$ or less.

In this connection, specifically, the density of tablet can be roughly estimated such as by the following calculation method. When the tablet has a cylindrical shape, density of the tablet is roughly estimated by using volume (A) roughly estimated from tablet thickness and tablet diameter of the tablet and tablet mass (B) and calculating (B) ÷ (A).

That is, in the case of a cylindrical tablet having a diameter of the tablet of L cm and thickness of the tablet of D cm, the volume (A) is $\{(L \div 2)^2 \times \pi \times D\}$. When mass of the tablet is regarded as B g, density of the tablet can be calculated by the following formula 1.

$$B/\{(L \div 2)^2 \times \pi \times D\} \qquad\qquad (\text{formula } 1)$$

For example, in the case of a cylindrical tablet having a tablet thickness of 0.36 cm and a tablet diameter (diameter) of 0.85 cm, the volume (A) becomes $(0.85 \div 2)^2 \times \pi \times 0.36 = 0.20428$ cm$^3$.

Accordingly, when the mass of the finally obtained tablet is, for example, 0.25 g, the density becomes

$$0.250 \text{ g} \div 0.20428 \text{ cm}^3 \doteqdot 1.22 \text{ g/cm}^3,$$

and therefore it would not be floatable in water which is larger than 1 g/cm$^3$.

In this case, when mass of the tablet is set, for example, to 200 mg by further enlarging the size of the cavity part, it would float since its density becomes 1 g/cm$^3$ or less.

The tablet of the invention comprises a filler. The filler means an additive agent which is mixed for the purpose of filling. The filler is at least one substance selected from sugar alcohols, sugars, cellulose derivatives and starches.

As the sugar alcohols, examples include mannitol, xylitol and the like.

As the sugars, examples include lactose, sucrose, fructose and the like.

As the cellulose derivative, examples include crystalline cellulose and the like.

As the starches, examples include corn starch and the like. Among these, mannitol, lactose and crystalline cellulose are preferable. As the mannitol, D-mannitol is preferable.

The content of the filler in the tablet can be optionally adjusted, but in general, it is preferably from 5% by mass to 95% by mass, and more preferably from 10% by mass to 80% by mass.

However, a substance such as METOLOSE which swells at the time of contact with a solvent is not suitable for the tablet of the invention since it causes a change in shape and swells and therefore the hardness is considerably lowered. In addition, there cannot be used a filler which is denatured by the heating temperature in removing the sublimation solid during the production process.

According to this specification, the term "exhibits a hydrophobic effect" means that it has a low affinity for water, namely a property of hardly dissolving in water or hardly mixing with water. The tablet of the invention can gradually release an ingredient which exhibits drug efficacy by comprising a component which exhibits a hydrophobic effect. As a result, duration of the effect relating to an ingredient which exhibits drug efficacy can be expected.

It is preferable to immerse the hollow tablet in a thermally melted component which exhibits a hydrophobic effect and then to solidify it by cooling, thereby contain the component in the tablet. Because of this, a fatty substance having a low melting point which is solid at room temperature is preferable as the component which exhibits a hydrophobic effect. The low melting point fatty substance has a melting point of preferably 40˚C to 100˚C, and more preferably 50˚C to 90˚C. In addition, a substance showing low viscosity when it is melted is preferable since it is easy to be immersed into the tablet.

As the component which exhibits a hydrophobic effect, examples include higher fatty acid glycerin esters and higher alcohol, waxes, organic acid which is not dissolved by gastric juice, and the like. Suspending and retention time of the tablet in water can be controlled by the kind of the component which exhibits a hydrophobic effect. In addition, in case that the tablet comprises a main drug component, releasing rate of the main drug component can be controlled by the kind of the component which exhibits a hydrophobic effect.

Examples of the higher fatty acid glycerin esters include stearic acid monoglyceride, hydrogenated castor oil, palmitic acid stearic acid monoglyceride, oleic acid monoglyceride, stearic acid mono-diglyceride and stearic acid oleic acid monoglyceride.

Examples of the higher alcohol include myristyl alcohol, cetyl alcohol, stearyl alcohol, 1-eicosanol, 1-docosanol, 1-tetracosanol, ceryl alcohol, octacosan-1-ol and 1-triacontanol.

Examples of the organic acid which is not dissolved by gastric juice include higher fatty acids such as stearic acid, decanoic acid, undecanoic acid, lauric acid, tridecanoic acid, myristic acid, pentadecanoic acid, palmitic acid and heptadecanoic acid.

From the viewpoint of permeability and from the viewpoint of sustained release effect, among these, stearic acid monoglyceride, hydrogenated castor oil, stearyl alcohol, cetyl alcohol and stearic acid are preferred. In the invention, the component to be used which exhibits a hydrophobic action which can be used can be optionally selected depending on the thermal stability of the component to be used for the filler and the thermal stability of the main drug component.

It is preferable that the content of the component which exhibits a hydrophobic effect in the tablet of the invention is optionally adjusted depending on the kind of the component, density of the tablet and the like, but in general, it is preferably from 5% by mass to 40% by mass, more preferably from 10% by mass to 30% by mass, preferably from 15% by mass to 25% by mass.

In the present specification, the term "floatable in water" means that it can exert buoyancy in a solvent such as water and can float for a prolonged period of time on the surface of liquid such as gastric juice. Floatable time of the tablet in water is preferably 1 hour or more, more preferably 3 hours or more, particularly preferably 5 hours or more, after the administration.

It is preferable that the tablet of the invention comprises a main drug component in a part other than the cavity part, namely the part constituting the tablet. By this, the main drug component can be released while the tablet is floatable in water.

According to the present specification, the term "main drug component" means a component which has a pharmacological activity. As the main drug component, it is not particularly limited, but in the case of using in a gastric floatable type tablet, specially the following drugs can be mentioned.

Examples include an agent for psychoneurosis such as barbitals, chloropromazine, levodopa, diazepam and imipramin; an analgesic antipyretic anti-inflammatory drug such as acetaminophen, aspirin, ibuprofen, ketoprofen and indometasin ; an antihistaminic such as diphenhydramine hydrochloride ; a β-blocker such as propranolol hydrochloride; a diuretic such as spironolactone, acetazolamide and furosemide; a hypotensive agent such as captopril and bunazosin hydrochloride : coronary vasodilator such as diltiazem hydrochloride and isosorbide nitrate ; a Ca antagonist such as nifedipine, nicardipine hydrochloride, nisoldipine and nitrendipine; an antilipidemic agent such as pravastatin ; an antitussive such as theophylline and codeine phosphate ; a digestive such as pepsin and diastase ; an antacid such as synthetic aluminum silicate and magnesium oxide ; an anti-ulcerogenic agent such as cimetidine ; a vitamin compound such as riboflavin ; various antibiotics such as of tetracycline system, penicillin system and cephem system ; a synthetic antibacterial agent such as ofloxacin and ciprofloxacin ; an antifungal agent such as tioconazole and griseofulvin; an anti-malignant tumor agent such as 5-FU , and the like.

Particularly, a drug which exerts direct effect on gastric site (e.g., acetohydroxamic acid), a drug which is thoroughly absorbed in the stomach or small intestine upper parts (e.g., ciprofloxacin and sotalol hydrochloride), a drug which is degraded by the intestinal juice or unstable in the intestinal juice (e.g., captopril) and a drug which is hardly dissolved in the intestinal juice or has poor solubility in the intestinal juice (e.g., diazepam and verapamil hydrochloride) are preferable.

In the case of a tablet which is floated on a paddy field, any substance which is generally used in the paddy field can be used as agricultural chemicals, and one or two or more of them may be used in combination. As such agriculturally active components, the following can be mentioned as an example.

Examples of an insecticide include CYAP, MPP, MEP, ECP, pirimiphos-methyl, etrimifos, diazinon, quinalphos, isoxathion, pyridafenthion, chlorpyrifos-methyl, chlorpyrifos, ECP, vamidothion, profenofos, marathon, PAP, dimethoate, formothion, thiometon, ethyl thiometon, phosalone, PMP, DMTP, prothiofos, sulprofos, pyraclofos, DDVP, monocrotophos, BRP, CVMP, dimethylvinphos, CVP, propaphos, acephate, isofenphos, salithion, DEP, EPN, ethion, NAC, MTMC, MIPC, BPMC, PHC, MPMC, XMC, ethiofencarb, bendiocarb, pirimicarb, carbosulfan, benfuracarb, thiodicarb, allethrin, resmethrin, permethrin, cypermethrin, cyhalothrin, cyfluthrin, fenpropathrin, tralomethrin, cycloprothrin, fenvalerate, flucythrinate, fluvalinate, etofenprox, pyrethrin, rotenone, nicotine sulfate, machine oil, rape seed oil, CPCBS, kelthane, chlorobenzilate, phenisobromolate, tetradifon, BPPS, chinomethionate, amitraz, benzomate, binapacryl, fenothiocarb, hexythiazox, fenbutatin oxide, dienochlor, polynactin complex, clofentezine, epizoepin, cartap, thiocyclam, bensultap, diflubenzuron, chlorfluazuron, buprofezin, BT and the like.

Examples of a bactericide include copper sulfate, calcium hydroxide, basic copper sulfate calcium, basic copper sulfate, basic copper chloride, cupric hydroxide, copper ammonium complex, oxine copper, nonylphenol copper sulfonate, DBEDC, copper terephtalate, sulfur, lime polysulfide, zineb, maneb, manzeb, amobam, polycarbamate, organic sulfur nickel salt, propineb, ziram, thiuram, milneb, captain, dichlorofluanid, TPN, fthalide, IBP, EDDP, triclofos methyl, pyrazophos, Fosetyl, thiophanate-methyl, benomyl, carbendazol, thiabendazole, iprodione, vinclozolin, procymidone, fluoroimide, oxycarboxin, mepronil, flutolanil, tecloftalam, trichlamide, pencycuron, metalaxyl, oxadixyl, triadimefon, bitertanol, triflumizole, fenarimol, triforine, blasticidin S, kasugamycin, polyoxin, validamycin A, streptomycin, oxytetracycline, mildiomycin, PCNB, hydroxyisoxazole, echlomezol, dazomet, methasulfocarb, zinc sulfate, triphenyltin hydroxide, MAF, MAFE, dithianon, benthiazole, phenazine oxide, CNA, DPC, dimethirimol, diclomezine, anilazine, probenazole, isoprothiolane, tricyclazole, pyroquilon, oxonic acid, guazatine, propamocarb hydrochloride, soybean lecithin and the like.

Examples of a herbicide include 2,4-D, MCP, MCPB, MCPP, triclopyr, phenothiol, clomeprop, naproanilide, fenoxaprop ethyl, fluazifop, CNP, chlomethoxynil, bifenox, MCC, IPC, phenmedipham, MBPMC, vernolate, benthiocarb, orthobencarb, esprocarb, molinate, dimepiperate, DCPA, alachlor, butachlor, pretilachlor, metolachlor, bromobutide, mefenacet, dymuron, bensulfuron methyl, simetryn, prometryn, dimethametryn, bentazon, oxadiazon, pyrazolate, pyrazoxyfen, benzofenap, trifluralin, piperophos, butamifos, bensulide, DCBN, ACN and the like.

Examples of a plant controlling agent include inabenfide, oxyethylenedocosanol, nicotinamide, benzylaminopurine and the like.

The content of the main drug component in the tablet varies depending on the kinds and the like of the selected filler, component which exhibits a hydrophobic effect and main drug component but, in general, is preferably from 5% by mass to 95% by mass, more preferably 10% by mass to 80% by mass, and particularly preferably 15% by mass to 70% by mass.

In addition to the above-mentioned components, in response to the necessity, a binder, a lubricant, a flavor, a disintegrant, a coloring agent, a sweetener, a corrigent, an antiseptic and the like may be optionally combined in the tablet of the invention.

In the present this specification, the term "sustained release effect" means that the drug is gradually released from the preparation. Since the tablet of the invention is floatable in water since it has a hollow structure and it is not disintegrated in water since it comprises a component which exhibits a hydrophobic effect, it can exert a sustained release effect by releasing the main drug component into water while floating and retaining for a prolonged period of time.

In addition, since the tablet of the invention particularly comprises a component which exhibits a hydrophobic effect in the stomach, it can exert a sustained release effect by releasing the main drug component while floating and retaining for a prolonged period of time without being disintegrated in the stomach. Because of this, a sustained release effect for continuing the drug effect can be exerted by applying to a drug wherein its acting region is the stomach, a drug which is absorbed quickly in the stomach and slowly in the intestinal tract, a drug of which absorption site (absorption window) is limited to an upper small intestine, a drug which is unstable in the intestinal environment, and the like.

The releasing mode can be optionally selected depending on the properties of the drug. As the releasing mode, specially, for example, a releasing mode in which releasing ratio of the drug after 1 hour is approximately 20% and its releasing ratio after 5 hours is 90% or more may be included. The releasing mode can be optionally adjusted based on the kinds and content of the filler, component which exhibits a hydrophobic effect and main drug component, density of the tablet, and the like.

The tablet of the invention can be produced by a method which includes at least the following steps (1) to (4):

(1) a step for preparing a core part including a sublimation solid;
(2) a step for preparing a dry coated tablet by forming a crust including at least one or more fillers selected from sugar alcohols, sugars, cellulose derivatives and starches on the outside of the core part in such a manner that the core part obtained by the step (1) is positioned at the center section;
(3) a step for obtaining a tablet having a hollow structure by removing the sublimation solid contained in the core part by heating the dry coated tablet obtained in the step (2); and
(4) a step for immersing the tablet having a hollow structure obtained in the step (3) in a component which exhibits a hydrophobic effect.

Each of the steps are described in the following.

(1) A step for preparing a core part comprising a sublimation solid

The step (1) is a step in which a core part is prepared by making a tablet from a sublimation solid. The core part is prepared by grinding a sublimation solid in a mortar using a pestle and then molding it compressively by a tablet making machine having appropriate sizes of a die and punches.

In the present specification, the term "sublimation solid" means a substance which has a melting point of 25°C or more and also shows a subliming property. Specially, examples include terpenes such as menthol, thymol and camphor, an aromatic hydrocarbon having a subliming property such as naphthalene, and the like.

When a gastric floating type oral sustained release preparation is prepared, terpenes such as menthol, thymol and camphor are preferable.

In addition, though the sublimation solid to be used can be optionally selected depending on the thermal stability of the component to be used in the filler and thermal stability of the main drug component, menthol is preferable among these. Also, both of 1-menthol and dl-menthol can be used as the menthol.

In the specification, the term "core part" means a partial structure obtained by subjecting the subliming substance to tablet making and the like. The core part containing the sublimation solid is removed by the heating treatment of step (3) after preparation of the crust in step (2). Accordingly, shape of the core part itself finally becomes the cavity part of the tablet relating to the invention.

Size and shape of the core part can be optionally selected depending on the size and shape of the desired cavity part. For example, when a disc-like core part having a diameter of 6 mm and a thickness of 1 mm is prepared, cavity part of the finally obtained tablet can also be made into a disc-like shape having a diameter of 6 mm and a thickness of 1 mm.

In case where a core part containing a subliming substance is prepared, it may be prepared by a direct powder compression method or a tablet may be prepared after granulation using a dry granulation method, a wet granulation method and the like, but the direct powder compression method is preferable from the viewpoint of productivity, handleability and convenience.

When the tablet is prepared by the direct powder compression method, as the tablet making machine, a generally used machine such as a rotary-type tablet making machine, and a single-type tablet making machine can be used. The tablet making pressure can be optionally adjusted depending on the subliming substance to be used, but in general, it is preferably 300 kg to 2000 kg and more preferably 100 kg to 1000 kg.

As the shape of the core part containing a sublimation solid, examples include a circle and various heteromorphic shapes having plane forms such as an oval, a flat oval and a square.

(2) A step for preparing a dry coated tablet by forming a crust comprising at least one or more fillers selected from sugar alcohols, sugars, cellulose derivatives and starches on the outside of the core part in such a manner that the core part obtained by the step (1) is positioned at the center section

The step (2) is a step for preparing a dry coated tablet by forming a crust containing a filler on the outside of the sublimation solid-containing core part prepared in the step (1). According to this specification, the term "crust" generally means the outer layer of the tablet. In the case of the tablet of the invention, this means the solid part positioned at around the cavity part. In this connection, the crust may comprise the main drug component.

Formation of the crust is carried out in the following manner. A part of the crust-constituting substance containing a filler is made into a tablet under a low tablet making pressure. When a main drug component is comprised in the tablet of the invention, the main drug component is mixed with the aforementioned crust-constituting substance. In this case, the low tablet making pressure is preferably 5 kg to 40 kg, more preferably 7 kg to 25 kg, and particularly preferably 10 kg to 15 kg. In addition, the part of the crust-constituting substance when the tablet is formed under a low tablet making pressure is preferably from 1/4 to 1/2 of the total amount of the crust-constituting substance, and more preferably 1/3 of the total amount.

Next, a dry coated tablet can be obtained by putting the core part obtained by the step (1) on the center section of the crust-constituting substance which is into a tablet under a low tablet making pressure, further packing remaining amount of the crust-constituting substance from the upper side and then subjecting the product to compression using a tablet making machine. In addition, the dry coated tablet of this step can also be produced using a continuous dry coated tablet making machine. The tablet making pressure in carrying out the compression can be optionally adjusted depending on the filler to be used, but is preferably 300 kg to 2000 kg and more preferably 500 kg to 1500 kg.

As the shape of the crust, for example, a circle and various heteromorphic shapes having plane forms such as an oval, a flat oval, a square and the like may be mentioned.

(3) A step for obtaining a tablet having a hollow structure by removing the sublimation solid contained in the core part by heating the dry coated tablet obtained in the step (2)

The step (3) is a step for removing the sublimation solid contained in the core part positioning inside of the crust of the dry coated tablet obtained by the step (2), by melting and subliming it through heating. By carrying out heating treatment of the dry coated tablet obtained by the step (2), the sublimation solid contained in the core part flows out and is sublimed into the outside moiety through inside of the crust and therefore the core part is removed.

As the heating method, examples include a method in which the dry coated tablet obtained by the step (2) is allowed to stand still in a heated oven.

The heating conditions can be optionally selected based on the thermal stability of the component to be used as the filler, thermal stability of the main drug component and properties of the sublimation solid and therefore they are not particularly limited, but the heating temperature is generally preferably 60˚C or more, more preferably 70˚C or more, particularly preferably 80˚C or more. In addition, the heating time is generally preferably 60 minutes to 180 minutes, more preferably 80 minutes to 120 minutes, and particularly preferably 90 minutes to 100 minutes.

Specifically, for example, when menthol is used in the core and removed under normal pressure, the heating temperature is generally preferably 50˚C or more, more preferably 70˚C or more, and particularly preferably 80˚C or more. In addition, when menthol is used in the core and removed under normal pressure, the heating time is generally preferably 60 minutes to 180 minutes, more preferably 60 minutes to 120 minutes, and particularly preferably 60 minutes to 90 minutes.

In addition, for example, in the case of a tablet which has a crust prepared using 150 mg of D-mannitol as the filler and contains a core part containing 75 mg of menthol in the center section of inside of the crust, a hollow tablet from which the core part is completely removed is obtained by heating at 80˚C for 1 hour.

(4) A step for immersing the tablet having a hollow structure obtained in the step (3) in a component which exhibits a hydrophobic effect.

The step (4) is a step for immersing the tablet having a hollow structure obtained by the step (3) in a previously heat-melted component which exhibits a hydrophobic effect. In addition, though the immersing time can be optionally set, it shows a sufficient effect within a short period of time. Specifically, it is generally preferably 30 seconds to 5 minutes, more preferably 1 minute to 3 minutes, and particularly preferably 1 minute. By passing through this step, the drug release rate is drastically changed and sustained release of a drug having instant solubility becomes possible.

The tablet immersed in a component which exhibits a hydrophobic effect is pulled out from the heat-melted component which exhibits a hydrophobic effect and cooled by leaving it at the melting point or less of the heat-melted component. The cooling temperature can be optionally selected, but it is preferable to leave it at room temperature from the viewpoint of convenience of handling. By cooling the tablet immersed in the component which exhibits a hydrophobic effect, the component which exhibits a hydrophobic effect and is permeated into the tablet is cooled and solidified.

As the cooling conditions, specifically, for example, when stearyl alcohol is used as the "component which exhibits a hydrophobic effect in the stomach", examples include a method in which the tablet pulled out from a heat-melted stearyl alcohol is allowed to stand still at room temperature for 10 seconds.

In addition, since the production method of the invention is extremely easy, the sublimation solid can be removed within a extremely short period of time and also completely, and sufficient floatable property can be added to the tablet. In addition, since the step for immersing a component which exhibits a hydrophobic effect in the stomach is also short, namely about 1 minute, it facilitates the production. By optionally selecting a component which exhibits a hydrophobic effect in the stomach, it becomes easy to prepare tablets having different sustained release effects.

[Examples]

The invention is described in the following with reference to examples and comparative examples, but the invention is not limited thereto.

[Example 1]

After 1-menthol (Kanto Chemical Co., Inc., special grade) was ground down using a mortar, a tablet of the core part was obtained by making the resulting substance into tablet under a pressure of 500 kg so as to have mass of 75 mg by a tablet making machine (HATA IRON WORKS CO., LTD, HT-AP-18-SSII), using a die having a diameter of 6 mm and flat-faced punches.

Separately from the tablet of the core part, a powder was prepared as a crust component by mixing 120 mg of mannitol (trade name Mannit-P, Towa Chemical Industry) and 30 mg of famotidine which were passed through a 850 μm sieve, and a 50 mg potion thereof was made into a tablet under a pressure of 15 kg by the tablet making machine (HATA IRON WORKS CO., LTD, HT-AP-18-SSII), using a die having a diameter of 8.5 mm and flat-faced punches.

The previously prepared tablet of the core part was put on the center section of the tablet prepared by making the crust component into tablet, the remaining 100 mg of the crust component was filled by covering the tablet of the core part, and then dry coated tablets having mass per one tablet of 225 mg were obtained by making it into tablet under a pressure of 1000 kg by the tablet making machine (HATA IRON WORKS CO., LTD, HT-AP-18-SSII), using a die having a diameter of 8.5 mm and flat-faced punches.

Thereafter, the thus obtained dry coated tablet was heated at 80˚C in an oven for 90 minutes to melt and sublime 1-menthol, thereby preparing a hollow tablet. When the density of the thus obtained tablet was calculated using the formula 1, it was $0.740 \pm 0.002$ g/cm$^3$.

After the thus obtained hollow tablet was immersed for 1 minute in hydrogenated castor oil (Trade name LubriWax 101, Freund Corporation) which was melted on a water bath of 90˚C, the tablet was quickly pulled out and then solidified by cooling at room temperature to obtain a tablet. When the density of the thus obtained tablet was calculated by the formula 1, it was $0.872 \pm 0.022$ g/cm$^3$.

[Example 2]

After 1-menthol (Kanto Chemical Co., Inc., special grade) was ground down using a mortar, a tablet of the core part was obtained by making the resulting substance into tablet under a pressure of 500 kg so as to have mass of 75 mg by a tablet making machine (HATA IRON WORKS CO., LTD, HT-AP-18-SSII), using a die having a diameter of 6 mm and flat-faced punches.

Separately from the tablet of the core part, a powder was prepared as a crust component by mixing 120 mg of mannitol (trade name Mannit-P, Towa Chemical Industry) and 30 mg of famotidine which were passed through a 850 μm sieve, and a 50 mg potion thereof was made into a tablet under a pressure of 15 kg by the tablet making machine (HATA IRON WORKS CO., LTD, HT-AP-18-SSII), using a die having a diameter of 8.5 mm and flat-faced punches.

The previously prepared tablet of the core part was put on the center section of the tablet prepared by making the crust component into tablet, the remaining 100 mg of the crust component was filled by covering the tablet of the core part, and then dry coated tablets having mass per one tablet of 225 mg were obtained by making it into tablet under a pressure of 1000 kg by the tablet making machine (HATA IRON WORKS CO., LTD, HT-AP-18-SSII), using a die having a diameter of 8.5 mm and flat-faced punches.

Thereafter, the thus obtained dry coated tablet was heated at 80˚C in an oven for 90 minutes to melt and sublime 1-menthol, thereby preparing a hollow tablet. After the thus obtained hollow tablet was immersed for 1 minute in stearyl alcohol (Trade name NAA-45, NIPPON OIL & FATS CO., LTD.) which was melted on a water bath of 90˚C, the tablet was quickly pulled out and then was solidified by cooling at room temperature to obtain a tablet. When the density of the thus obtained tablet was calculated by the formula 1, it was $0.827 \pm 0.021$ g/cm$^3$.

[Example 3]

After 1-menthol (Kanto Chemical Co., Inc., special grade) was ground down using a mortar, a tablet of the core part was obtained by making the resulting substance into tablet under a pressure of 500 kg so as to have mass of 75 mg by a tablet making machine (HATA IRON WORKS CO., LTD, HT-AP-18-SSII), using a die having a diameter of 6 mm and flat-faced punches.

Separately from the tablet of the core part, a powder was prepared as a crust component by mixing 120 mg of mannitol (trade name Mannit-P, Towa Chemical Industry) and 30 mg of famotidine which were passed through a 850 $\mu$m sieve, and a 50 mg potion thereof was made into a tablet under a pressure of 15 kg by the tablet making machine (HATA IRON WORKS CO., LTD, HT-AP-18-SSII), using a die having a diameter of 8.5 mm and flat-faced punches.

The previously prepared tablet of the core part was put on the center section of the tablet prepared by making the crust component into tablet, the remaining 100 mg of the crust component was filled by covering the tablet of the core part, and then dry coated tablets having mass per one tablet of 225 mg were obtained by making it into tablet under a pressure of 1000 kg by the tablet making machine (HATA IRON WORKS CO., LTD, HT-AP-18-SSII), using a die having a diameter of 8.5 mm and flat-faced punches.

Thereafter, the thus obtained dry coated tablet was heated at 80˚C in an oven for 90 minutes to melt and sublime 1-menthol, thereby preparing a hollow tablet. After the thus obtained hollow tablet was immersed for 1 minute in stearic acid monoglyceride (Trade name MGS-AMV, NIHON SURFACTANT KOGYO K.K.) which was melted on a water bath of 90˚C, the tablet was quickly pulled out and then was solidified by cooling at room temperature to obtain a tablet. When the density of the thus obtained tablet was calculated by the formula 1, it was $0.841 \pm 0.034$ g/cm$^3$.

[Example 4]

After 1-menthol (Kanto Chemical Co., Inc., special grade) was ground down using a mortar, a tablet of the core part was obtained by making the resulting substance into tablet under a pressure of 500 kg so as to have mass of 75 mg by a tablet making machine (HATA IRON WORKS CO., LTD, HT-AP-18-SSII), using a die having a diameter of 6 mm and flat-faced punches.

Separately from the tablet of the core part, a powder was prepared as a crust component by mixing 120 mg of mannitol (trade name Mannit-P, Towa Chemical Industry) and 30 mg of famotidine which were passed through a 850 $\mu$m sieve, and a 50 mg potion thereof was made into a tablet under a pressure of 15 kg by the tablet making machine (HATA IRON WORKS CO., LTD, HT-AP-18-SSII), using a die having a diameter of 8.5 mm and flat-faced punches.

The previously prepared tablet of the core part was put on the center section of the tablet prepared by making the crust component into tablet, the remaining 100 mg of the crust component was filled by covering the tablet of the core part, and then dry coated tablets having mass per one tablet of 225 mg were obtained by making it into tablet under a pressure of 1000 kg by the tablet making machine (HATA IRON WORKS CO., LTD, HT-AP-18-SSII), using a die having a diameter of 8.5 mm and flat-faced punches.

Thereafter, the thus obtained dry coated tablet was heated at 80˚C in an oven for 90 minutes to melt and sublime 1-menthol, thereby preparing a hollow tablet. After the thus obtained hollow tablet was immersed for 1 minute in a 1:1 by mass mixture of stearic acid monoglyceride (Trade name MGS-AMV, NIHON SURFACTANT KOGYO K.K.) and hydrogenated castor oil (Trade name LubriWax 101, Freund Corporation), which were melted on a water bath of 90˚C, the tablet was quickly pulled out and then was solidified by cooling at room temperature to obtain a tablet. When the density of the thus obtained tablet was calculated by the formula 1, it was $0.818 \pm 0.008$ g/cm$^3$.

[Example 5]

After 1-menthol (Kanto Chemical Co., Inc., special grade) was ground down using a mortar, a tablet of the core part was obtained by making the resulting substance into tablet under a pressure of 500 kg so as to have mass of 75 mg by a tablet making machine (HATA IRON WORKS CO., LTD, HT-AP-18-SSII), using a die having a diameter of 6 mm and flat-faced punches.

Separately from the tablet of the core part, a powder was prepared as a crust component by mixing 120 mg of mannitol (trade name Mannit-P, Towa Chemical Industry) and 30 mg of famotidine which were passed through a 850

μm sieve, and a 50 mg potion thereof was made into a tablet under a pressure of 15 kg by the tablet making machine (HATA IRON WORKS CO., LTD, HT-AP-18-SSII), using a die having a diameter of 8.5 mm and flat-faced punches.

The previously prepared tablet of the core part was put on the center section of the tablet prepared by making the crust component into tablet, the remaining 100 mg of the crust component was filled by covering the tablet of the core part, and then dry coated tablets having mass per one tablet of 225 mg were obtained by making it into tablet under a pressure of 1000 kg by the tablet making machine (HATA IRON WORKS CO., LTD, HT-AP-18-SSII), using a die having a diameter of 8.5 mm and flat-faced punches.

Thereafter, the thus obtained dry coated tablet was heated at 80°C in an oven for 90 minutes to melt and sublime 1-menthol, thereby preparing a hollow tablet. After the thus obtained hollow tablet was immersed for 1 minute in a 1:1 by mass mixture of stearyl alcohol (Trade name NAA-45, NIPPON OIL & FATS CO., LTD.) and hydrogenated castor oil (Trade name LubriWax 101, Freund Corporation) which were melted on a water bath of 90°C, the obtained tablet was quickly pulled out and solidified by cooling at room temperature to obtain a tablet. When the density of the thus obtained tablet was calculated by the formula 1, it was $0.857 \pm 0.004$ g/cm$^3$.

[Example 6]

After 1-menthol (Kanto Chemical Co., Inc., special grade) was ground down using a mortar, a tablet of the core part was obtained by making the resulting substance into tablet under a pressure of 500 kg so as to have mass of 75 mg by a tablet making machine (HATA IRON WORKS CO., LTD, HT-AP-18-SSII), using a die having a diameter of 6 mm and flat-faced punches.

Separately from the tablet of the core part, a powder was prepared as a crust component by mixing 60 mg of mannitol (trade name Mannit-P, Towa Chemical Industry) and 90 mg of famotidine which were passed through a 850 μm sieve, and a 50 mg potion thereof was made into a tablet under a pressure of 15 kg by the tablet making machine (HATA IRON WORKS CO., LTD, HT-AP-18-SSII), using a die having a diameter of 8.5 mm and flat-faced punches.

The previously prepared tablet of the core part was put on the center section of the tablet prepared by making the crust component into tablet, the remaining 100 mg of the crust component was filled by covering the tablet of the core part, and then dry coated tablets having mass per one tablet of 225 mg were obtained by making it into tablet under a pressure of 800 kg by the tablet making machine (HATA IRON WORKS CO., LTD, HT-AP-18-SSII), using a die having a diameter of 8.5 mm and flat-faced punches.

Thereafter, the thus obtained dry coated tablet was heated at 80°C in an oven for 90 minutes to melt and sublime 1-menthol, thereby preparing a hollow tablet. After the thus obtained hollow tablet was immersed for 1 minute in a 1:1 by mass mixture of stearic acid monoglyceride (Trade name MGS-AMV, NIHON SURFACTANT KOGYO K.K.) and hydrogenated castor oil (Trade name LubriWax 101, Freund Corporation) which were melted on a water bath of 90°C, the obtained tablet was quickly pulled out and then was solidified by cooling at room temperature to obtain a tablet. When the density of the thus obtained tablet was calculated by the formula 1, it was $0.853 \pm 0.015$ g/cm$^3$.

[Example 7]

After 1-menthol (Kanto Chemical Co., Inc., special grade) was ground down using a mortar, a tablet of the core part was obtained by making the resulting substance into tablet under a pressure of 500 kg so as to have mass of 75 mg by a tablet making machine (HATA IRON WORKS CO., LTD, HT-AP-18-SSII), using a die having a diameter of 6 mm and flat-faced punches.

Separately from the tablet of the core part, a powder was prepared as a crust component by mixing 30 mg of mannitol (trade name Mannit-P, Towa Chemical Industry) and 120 mg of famotidine which were passed through a 850 μm sieve, and a 50 mg potion thereof was made into a tablet under a pressure of 15 kg by the tablet making machine (HATA IRON WORKS CO., LTD, HT-AP-18-SSII), using a die having a diameter of 8.5 mm and flat-faced punches.

The previously prepared tablet of the core part was put on the center section of the tablet prepared by making the crust component into tablet, the remaining 100 mg of the crust component was filled by covering the tablet of the core part, and then dry coated tablets having mass per one tablet of 225 mg were obtained by making it into tablet under a pressure of 800 kg by the tablet making machine (HATA IRON WORKS CO., LTD, HT-AP-18-SSII), using a die having a diameter of 8.5 mm and flat-faced punches.

Thereafter, the thus obtained dry coated tablet was heated at 80°C in an oven for 90 minutes to melt and sublime 1-menthol, thereby preparing a hollow tablet. After the thus obtained hollow tablet was immersed for 1 minute in a 1:1 by mass mixture of stearic acid monoglyceride (Trade name MGS-AMV, NIHON SURFACTANT KOGYO K.K.) and hydrogenated castor oil (Trade name LubriWax 101, Freund Corporation), which were melted on a water bath of 90°C, the obtained tablet was quickly pulled out and then was solidified by cooling at room temperature to obtain a tablet. When the density of the thus obtained tablet was calculated by the formula 1, it was $0.856 \pm 0.007$ g/cm$^3$.

Next, by showing reference examples and test examples, usefulness of the invention is shown.

(Reference Example 1)

After 1-menthol (Kanto Chemical Co., Inc., special grade) was ground down using a mortar, a tablet of the core part was obtained by making the resulting substance into tablet under a pressure of 500 kg so as to have mass of 75 mg using a tablet making machine (HATA IRON WORKS CO., LTD, HT-AP-18-SSII), a die having a diameter of 6 mm and flat-faced punches.

Separately from the tablet of the core part, 50 mg of mannitol (trade name Mannit-P, Towa Chemical Industry) as a crust component which were passed through a 850 μm sieve was made into a tablet under a pressure of 10 kg using the tablet making machine (HATA IRON WORKS CO., LTD, HT-AP-18-SSII), a die having a diameter of 8.5 mm and flat-faced punches.

The previously prepared tablet of the core part was put on the center section of the tablet prepared by making the crust component into tablet, 100 mg of mannitol (trade name Mannit-P, Towa Chemical Industry) was filled by covering the tablet of the core part, and then a dry coated tablet having mass per one tablet of 225 mg was obtained by making the product into tablet under a pressure of 600 kg by the tablet making machine (HATA IRON WORKS CO., LTD, HT-AP-18-SSII), using a die having a diameter of 8.5 mm and flat-faced punches. Thereafter, 1-menthol was melted and sublimed by heating the dry coated tablet in an oven of 80˚C.

(Reference Example 2)

After 1-menthol (Kanto Chemical Co., Inc., special grade) was ground down using a mortar, a tablet of the core part was obtained by making the resulting subject into tablet under a pressure of 500 kg so as to have mass of 75 mg by a tablet making machine (HATA IRON WORKS CO., LTD, HT-AP-18-SSII), using a die having a diameter of 6 mm and flat-faced punches.

Separately from the tablet of the core part, 50 mg of crystalline cellulose (trade name CEOLUS PH-102, Asahi Kasei Chemicals Corporation) as a crust component was made into a tablet under a pressure of 20 kg by the tablet making machine (HATA IRON WORKS CO., LTD, HT-AP-18-SSII), using a die having a diameter of 8.5 mm and flat-faced punches.

The previously prepared tablet of the core part was put on the center section of the tablet prepared by making the crust component into tablet, 100 mg of crystalline cellulose (trade name CEOLUS PH-102, Asahi Kasei Chemicals Corporation) was filled by covering the tablet of the core part, and then a dry coated tablet having mass per one tablet of 225 mg was obtained by making the product into tablet under a pressure of 500 kg by the tablet making machine (HATA IRON WORKS CO., LTD, HT-AP-18-SSII), using a die having a diameter of 8.5 mm and flat-faced punches. Thereafter, 1-menthol was melted and sublimed by heating said dry coated tablet in an oven of 80˚C.

(Reference Example 3)

After 1-menthol (Kanto Chemical Co., Inc., special grade) was ground down using a mortar, a tablet of the core part was obtained by making the resulting substance into tablet under a pressure of 500 kg so as to have mass of 75 mg using a tablet making machine (HATA IRON WORKS CO., LTD, HT-AP-18-SSII), a die having a diameter of 6 mm and flat-faced punches.

Separately from the tablet of the core part, 50 mg of lactose (200 M, Fonterra) as a crust component was made into a tablet under a pressure of 12 kg by the tablet making machine (HATA IRON WORKS CO., LTD, HT-AP-18-SSII), using a die having a diameter of 8.5 mm and flat-faced punches.

The previously prepared tablet of the core part was put on the center section of the tablet prepared by making the crust component into tablet, 100 mg of lactose (200 M, Fonterra) was filled by covering the tablet of the core part, and then a dry coated tablet mass per one tablet of 225 mg was obtained by making the product into tablet under a pressure of 750 kg by the tablet making machine (HATA IRON WORKS CO., LTD, HT-AP-18-SSII), using a die having a diameter of 8.5 mm and flat-faced punches. Thereafter, 1-menthol was melted and sublimed by heating the dry coated tablet in an oven of 80˚C.

(Reference Example 4)

A tablet of a core part was obtained by grinding down 1-menthol, (Kanto Chemical Co., Inc., special grade) using a mortar and then making it into a tablet under a pressure of 500 kg so as to have mass of 75 mg by a tablet making machine (HATA IRON WORKS CO., LTD, HT-AP-18-SSII), using a die having a diameter of 6 mm and flat-faced punches. Separately from the tablet of the core part, 50 mg of hypromellose (trade name METOLOSE 90SH-4000SR, Shin-Etsu Chemical Co., Ltd.) as a crust component was made into a tablet under a pressure of 25 kg by the tablet making machine (HATA IRON WORKS CO., LTD, HT-AP-18-SSII), using a die having a diameter of 8.5 mm and flat-faced punches.

The previously prepared tablet of the core part was put on the center section of the tablet prepared by making the crust component into tablet, 100 mg of hypromellose (trade name METOLOSE 90SH-4000SR, Shin-Etsu Chemical Co., Ltd.) was filled by covering the tablet of the core part, and then a dry coated tablet having mass per one tablet of 225 mg was obtained by making the product into tablet under a pressure of 900 kg by the tablet making machine (HATA IRON WORKS CO., LTD, HT-AP-18-SSII), using a die having a diameter of 8.5 mm and flat-faced punches. Thereafter, 1-menthol was melted and sublimed by heating said dry coated tablet in an oven of 80˚C.

(Reference Example 5)

A tablet of a core part was obtained by grinding down 1-menthol, (Kanto Chemical Co., Inc., special grade) using a mortar and then making it into a tablet under a pressure of 500 kg so as to have mass of 75 mg by a tablet making machine (HATA IRON WORKS CO., LTD, HT-AP-18-SSII), using a die having a diameter of 6 mm and flat-faced punches.

Separately from the tablet of the core part, 50 mg of an aminoalkyl methacrylate copolymer (trade name Eudragit RS PO, Degussa) as a crust component was made into a tablet under a pressure of 15 kg by the tablet making machine (HATA IRON WORKS CO., LTD, HT-AP-18-SSII), using a die having a diameter of 8.5 mm and flat-faced punches.

The previously prepared tablet of a core part was put on the center section of the tablet prepared by making the crust component into tablet, 100 mg of the aminoalkyl methacrylate copolymer (trade name Eudragit RS PO, Degussa) was filled by covering the tablet of the core part, and then a dry coated tablet having mass per one tablet of 225 mg was obtained by making the product into tablet under a pressure of 1200 kg by the tablet making machine (HATA IRON WORKS CO., LTD, HT-AP-18-SSII), using a die having a diameter of 8.5 mm and flat-faced punches. Thereafter, 1-menthol was melted and sublimed by heating said dry coated tablet in an oven of 80˚C.

(Comparative Example 1)

A tablet of a core part was obtained by grinding down 1-menthol (Kanto Chemical Co., Inc., special grade) using a mortar and then making it into a tablet under a pressure of 500 kg so as to have mass of 75 mg by a tablet making machine (HATA IRON WORKS CO., LTD, HT-AP-18-SSII), using a die having a diameter of 6 mm and flat-faced punches.

Separately from the tablet of the core part, a powder was prepared by mixing 120 mg of mannitol (trade name Mannit-P, Towa Chemical Industry) and 30 mg of famotidine passed through a 850 $\mu$m sieve as the crust component, and a 50 mg portion thereof was made into a tablet under a pressure of 15 kg by the tablet making machine (HATA IRON WORKS CO., LTD, HT-AP-18-SSII), using a die having a diameter of 8.5 mm and flat-faced punches.

The previously prepared tablet of the core part was put on the center section of the tablet prepared by making the crust component into tablet, 100 mg of the remaining crust component was filled by covering the tablet of the core part, and then a dry coated tablet having mass per one tablet of 225 mg was obtained by making the product into tablet under a pressure of 1000 kg by the tablet making machine (HATA IRON WORKS CO., LTD, HT-AP-18-SSII), using a die having a diameter of 8.5 mm and flat-faced punches. Thereafter, a hollow tablet was prepared by melting and subliming 1-menthol by healing the dry coated tablet in an oven of 80˚C.

(Test Example 1) Ease of production of hollow structure

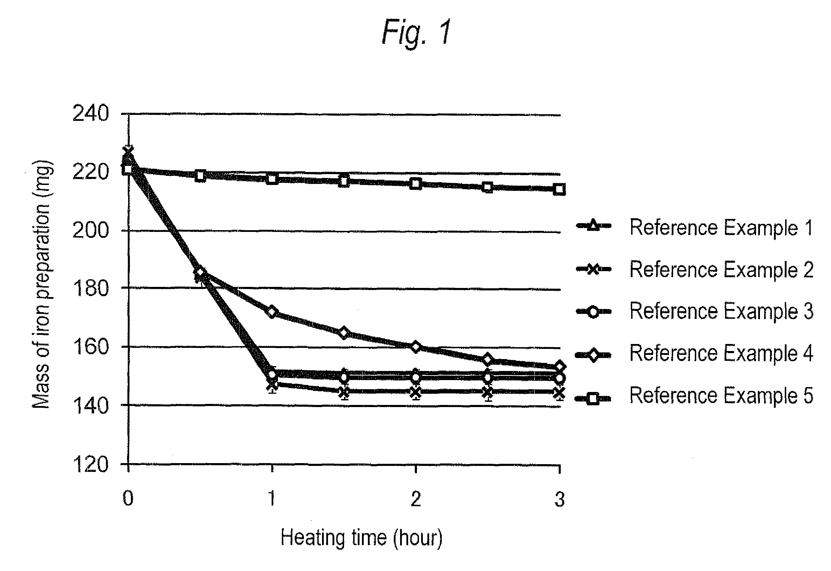

Fig. 1 shows changes in mass of the dry coated tablets of Reference Examples 1 to 5 when they were heated in an oven of 80˚C. In the case where mannitol (Reference Example 1), crystalline cellulose (Reference Example 2) or lactose (Reference Example 3) was used for the crust, since 1-menthol was easily removed, it was able to prepare hollow tablets. When hypromellose (Reference Example 4) was used for the crust, it took time for removing 1-menthol, and furthermore since hypromellose was slightly swelled in carrying out subliming, the crust became brittle and it was not practical.

When Eudragit RSPO was used in the crust (Reference Example 5), since glass transition point of Eudragit RSPO was lower than 80˚C of the oven, it caused softening and 1-menthol was hardly removed due to blocking of the escape route of 1-menthol and therefore the hollow tablet could not be prepared.

(Test Example 2) Dissolution test and floatability test

Using 900 ml of 0.01 M hydrochloric acid as the test liquid, the dissolution test was carried out using the paddle method at 100 revolutions per minutes. After 1, 2, 3, 4, 5 or 6 hours after commencement of the dissolution test, sampling was carried out through a membrane filter having a pore size of 0.45 $\mu$m, and dissolution rate was calculated by a liquid chromatography under the following conditions. The floatability test was visually confirmed at 30 minute intervals after commencement of the test.

Test conditions for liquid chromatography

Column: Inertsil ODS-3,4.6 mm in inner diameter, 250 mm in length, 5 $\mu$m in particle diameter, manufactured by GL Science
Mobile phase: 20 mM sodium dihydrogenphosphate aqueous solution/methanol for liquid chromatography mixed liquid (4:1)
Detector: Absorptiometer for ultraviolet and visible region (measuring wavelength: 254 nm)

Results of the floatability test are shown in Table 1. As shown in Table 1, Comparative Example 1 in which the step 4 and step 5 were not carried out floated just after commencement of the test, but it disintegrated and precipitated after several tens of seconds since mannitol Well dissolves in water. On the other hand, Examples to 7 in which the step 4 and step 5 were carried out floated until 6 hours after commencement of the test. This is expected that the tablets of Examples 1 to 7 float and remain in the stomach for a prolonged period of time. In this connection, the tablet from which the core part was not removed did not float.

[Table 1]

| Elapsed time (hr) | Ex. 1 | Ex. 2 | Ex. 3 | Ex. 4 | Ex. 5 | Ex. 6 | Ex. 7 | Comp. Ex. 1 |
|---|---|---|---|---|---|---|---|---|
| 0.5 | ○ | ○ | ○ | ○ | ○ | ○ | ○ | △* |
| 1 | ○ | ○ | ○ | ○ | ○ | ○ | ○ | X |
| 1.5 | ○ | ○ | ○ | ○ | ○ | ○ | ○ | X |
| 2 | ○ | ○ | ○ | ○ | ○ | ○ | ○ | X |
| 2.5 | ○ | ○ | ○ | ○ | ○ | ○ | ○ | X |
| 3 | ○ | ○ | ○ | ○ | ○ | ○ | ○ | X |
| 3.5 | ○ | ○ | ○ | ○ | ○ | ○ | ○ | X |
| 4 | ○ | ○ | ○ | ○ | ○ | ○ | ○ | X |
| 4.5 | ○ | ○ | ○ | ○ | ○ | ○ | ○ | X |
| 5 | ○ | ○ | ○ | ○ | ○ | ○ | ○ | X |
| 5.5 | ○ | ○ | ○ | ○ | ○ | ○ | ○ | X |
| 6 | ○ | ○ | ○ | ○ | ○ | ○ | ○ | X |
| ○: floatable X: no floatability △*: no suspension after several tens of seconds due to disintegration | | | | | | | | |

Results of the dissolution test are shown in Table 2. The dissolution rate was calculated by "amount of main drug component released from tablet at each dissolution time" ÷ "main drug component content in one tablet" × 100. In this connection, since the main drug component, famotidine, used in this test was degraded under acidic conditions, the "amount of main drug component released from tablet at each dissolution time" was calculated by adding up the amount of famotidine and the amount of its degradation products.

As shown in Table 2, Comparative Example 1 in which the step 4 and step 5 were not carried out did not show sustained release property because the tablet was immediately disintegrated. Contrary to this, Examples showed various degrees of sustained release property, It is considered that this difference in sustained release property is controlled by the difference in water solubility of the wax components to be permeated.

[Table 2]

| Dissolution time (hr) | 0.5 | 1 | 2 | 3 | 4 | 5 | 6 |
|---|---|---|---|---|---|---|---|
| Comparative Example 1 | 101 | 102 | 102 | 102 | 102 | 102 | 103 |
| Example 1 | 2 | 3 | 6 | 9 | 11 | 14 | 16 |
| Example 2 | 6 | 10 | 20 | 29 | 38 | 46 | 54 |
| Example 3 | 30 | 41 | 57 | 72 | 84 | 89 | 92 |
| Example 4 | 10 | 16 | 27 | 38 | 47 | 56 | 65 |
| Example 5 | 3 | 6 | 11 | 15 | 19 | 23 | 26 |
| Example 6 | 8 | 13 | 21 | 28 | 33 | 38 | 43 |
| Example 7 | 2 | 4 | 6 | 8 | 10 | 12 | 13 |
| Unit: % | | | | | | | |

While the present invention has been described in detail and with reference to specific embodiments thereof, it will be apparent to one skilled in the art that various changes and modifications can be made therein without departing from the spirit and scope thereof. This application is based on Japanese patent application filed July 6, 2009 (Japanese Patent Application No. 2009-159804) and Japanese patent application filed November 2, 2009 (Japanese Patent Application No. 2009-251715), and the contents thereof are herein incorporated by reference.

Industrial Applicability

According to the invention, it is useful because a tablet having high flexibility which renders possible sustainable release of a drug by suspending and retaining in water can be obtained by a convenient production method. The tablet of the invention can be applied to a gastric retaining preparation, a water quality clarification agent, a floatable agricultural chemical preparation, a floatable chlorine agent such as for pool use, a floatable cleaner such as for pool use, a floatable algae controlling agent such as for pool use, a floatable gradually dissolving type bathing agent, a floatable feeding agent for aquarium fish use, a floatable fishing bait agent and the like.

**Claims**

1.  A water floatable tablet, which has a hollow structure having a cavity part in the center section and comprises at least one filler selected from sugar alcohols, sugars, cellulose derivatives and starches and a component which exhibits a hydrophobic effect.

2.  A water floatable tablet, which is obtainable by a production method comprising at least the following steps (1) to (4):

    (1) a step of preparing a core part comprising a sublimation solid;
    (2) a step of preparing a dry coated tablet by forming a crust comprising at least one or more fillers selected from sugar alcohols, sugars, cellulose derivatives and starches on the outside of the core part in such a manner that the core part obtained by the step (1) is positioned at the center section;
    (3) a step of obtaining a tablet having a hollow structure by removing the sublimation solid comprised in the core part by heating the dry coated tablet obtained in the step (2); and
    (4) a step of immersing the tablet having a hollow structure obtained in the step (3) in a component which exhibits a hydrophobic effect.

3.  The tablet according to claim 1 or 2, wherein the component which exhibits a hydrophobic effect is a component which shows the hydrophobic effect in the stomach.

4.  The tablet according to any one of claims 1 to 3, which has a density of 1 g/cm$^3$ or less.

5.  The tablet according to any one of claims 1 to 4, which comprises a main drug component is comprised in a part other than the cavity part.

6. The tablet according to any one of claims 1 to 5, which has a sustained release effect.

7. The tablet according to any one of claims 1 to 6, wherein the filler is at least one substance selected from mannitol, crystalline cellulose, lactose and a mixture thereof.

8. The tablet according to any one of claims 1 to 7, wherein the component which exhibits a hydrophobic effect is at least one of a higher alcohol and a higher fatty acid glycerin ester.

9. The tablet according to any one of claims 1 to 8, wherein the component which exhibits a hydrophobic effect is at least one substance selected from stearyl alcohol, cetyl alcohol, hydrogenated castor oil and stearic acid monoglyceride.

10. A method for producing a water floatable tablet having a hollow structure, which comprises at least the following steps:

   (1) a step of preparing a core part comprising a sublimation solid;
   (2) a step of preparing a dry coated tablet by forming a crust comprising at least one or more fillers selected from sugar alcohols, sugars, cellulose derivatives and starches on the outside of the core part in such a manner that the core part obtained by the step (1) is positioned at the center section;
   (3) a step of obtaining a tablet having a hollow structure by removing the sublimation solid comprised in the core part by heating the dry coated tablet obtained in the step (2); and
   (4) a step of immersing the tablet having a hollow structure obtained in the step (3) in a component which exhibits a hydrophobic effect.

11. The production method according to claim 10, wherein the component which exhibits a hydrophobic effect is a component which shows the hydrophobic effect in the stomach.

12. The production method according to claim 10 or 11, wherein the sublimation solid is at least one of terpenes and an aromatic hydrocarbon having subliming property.

13. The production method according to claim 12, wherein the terpenes are at least one substance selected from menthol, thymol and camphor.

14. The production method according to any one of claims 10 to 13, wherein a main drug component is comprised in the crust part in the step (2).

15. The production method according to any one of claims 10 to 14, wherein the filler is at least one substance selected from mannitol, crystalline cellulose, lactose and a mixture thereof.

16. The production method according to any one of claims 10 to 15, wherein the component which exhibits a hydrophobic effect is at least one of a higher alcohol and a higher fatty acid glycerin ester.

17. The production method according to any one of claims 10 to 16, wherein the component which exhibits a hydrophobic effect is at least one substance selected from stearyl alcohol, cetyl alcohol, hydrogenated castor oil and stearic acid monoglyceride.

18. A method for retaining a tablet in the stomach by using the tablet according to any one of claims 1 to 9.

19. A method for sustainably releasing a drug in the stomach by using the tablet according to any one of claims 1 to 9.

Fig. 1

EP 2 457 561 A1

Fig. 2

EP 2 457 561 A1

Fig. 3

| | |
|---|---|
| **INTERNATIONAL SEARCH REPORT** | International application No.<br>PCT/JP2010/061430 |

**A. CLASSIFICATION OF SUBJECT MATTER**

*A61K9/20*(2006.01)i, *A61K31/426*(2006.01)i, *A61K47/08*(2006.01)i, *A61K47/10* (2006.01)i, *A61K47/14*(2006.01)i, *A61K47/26*(2006.01)i, *A61K47/36*(2006.01)i, *A61K47/38*(2006.01)i, *A61K47/44*(2006.01)i, *A61P1/04*(2006.01)i

According to International Patent Classification (IPC) or to both national classification and IPC

**B. FIELDS SEARCHED**

Minimum documentation searched (classification system followed by classification symbols)
A61K9/00-9/72, A61K47/080-47/48, A61K31/426, A61P1/04

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2010 |
| Kokai Jitsuyo Shinan Koho | 1971–2010 | Toroku Jitsuyo Shinan Koho | 1994–2010 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2007-210986 A (KAO CORP.),<br>23 August 2007 (23.08.2007),<br>claims<br>(Family: none) | 1-18 |
| A | WO 2008/062440 A2 (PANACEA BIOTEC LTD.),<br>29 May 2008 (29.05.2008),<br>claims<br>& EP 2068844 A2      & KR 2009065524 A<br>& NO 200900807 A      & AU 2007323018 A1<br>& CA 2661172 A1      & CN 101511346 A<br>& MX 2009002371 A1      & US 2010/015224 A1<br>& JP 2010502591 A | 1-18 |

☒ Further documents are listed in the continuation of Box C.      ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| | |
|---|---|
| Date of the actual completion of the international search<br>15 September, 2010 (15.09.10) | Date of mailing of the international search report<br>28 September, 2010 (28.09.10) |
| Name and mailing address of the ISA/<br>Japanese Patent Office | Authorized officer |
| Facsimile No. | Telephone No. |

Form PCT/ISA/210 (second sheet) (July 2009)

**EP 2 457 561 A1**

<table>
<tr><td colspan="2" align="center">**INTERNATIONAL SEARCH REPORT**</td><td>International application No.<br><br>PCT/JP2010/061430</td></tr>
</table>

C (Continuation).    DOCUMENTS CONSIDERED TO BE RELEVANT

| Category* | Citation of document, with indication, where appropriate, of the relevant passages | Relevant to claim No. |
|---|---|---|
| A | JP 05-024018 A  (NIPPON CEMENT KABUSHIKI KAISHA),<br>02 February 1993 (02.02.1993),<br>claims; examples<br>(Family: none) | 1-18 |

Form PCT/ISA/210 (continuation of second sheet) (July 2009)

EP 2 457 561 A1

**INTERNATIONAL SEARCH REPORT**

| International application No. |
|---|
| PCT/JP2010/061430 |

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 18 - 19
   because they relate to subject matter not required to be searched by this Authority, namely:
   Claims 18 - 19 pertain to the inventions concerning a method for retention of a tablet in the stomach using the tablet and a method for continuous release of a drug in the stomach.   These inventions are relevant to methods for treatment of the human body by therapy,  (continued to extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

22

**INTERNATIONAL SEARCH REPORT**

International application No.

PCT/JP2010/061430

Continuation of Box No.II-1 of continuation of first sheet(2)

and thus relate to a subject on which this International Searching Authority is not required to carry out a search under the provision of PCT Rule 39.1(iv).

Form PCT/ISA/210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007131591 A
- JP 2005532985 T
- JP 2002370970 A
- WO 955809 A
- JP 2005132803 A
- JP 2001500879 T
- JP 2008528636 T
- JP 6024959 A
- JP 2009040787 A
- JP 62283919 A
- JP 62195323 A
- JP 62207209 A
- JP 2009541777 T

- JP 1016715 A
- JP 2005112825 A
- JP 61043108 A
- WO 916281 A
- JP 64000030 A
- JP 1224311 A
- JP 2250822 A
- JP 5255067 A
- WO 00040085 A
- JP 10087477 A
- JP 2009501777 T
- JP 2009159804 A
- JP 2009251715 A

**Non-patent literature cited in the description**

- *European Journal of Pharmaceutics and Biopharmaceutics,* 2004, vol. 57, 235-243